Europäisches Patentamt

European Patent Office

Office européen des brevets

(11) Publication number: **0 027 694**
**A1**

## (12) EUROPEAN PATENT APPLICATION

(21) Application number: **80303465.1**

(22) Date of filing: **01.10.80**

(51) Int. Cl.³: **C 02 F 3/34**
**C 12 N 1/38, C 05 F 13/00**

(30) Priority: **22.10.79 US 86934**

(43) Date of publication of application:
**29.04.81 Bulletin 81/17**

(84) Designated Contracting States:
**AT BE CH DE FR GB IT LI LU NL SE**

(71) Applicant: **BIOHUMUS, INC.**
**Box 13**
**Holtville California 92250(US)**

(72) Inventor: **Starr, Jerry**
**947A Keffer Road**
**Holtville, California 92250(US)**

(74) Representative: **Wilson, Nicholas Martin et al,**
**WITHERS & ROGERS 4 Dyer's Buildings Holborn**
**London. EC1N 2JT(GB)**

(54) **Composition and method for stimulation of aerobic bacteria.**

(57) Composition for stimulation of aerobic bacterial from combination of triacontanol, B vitamins or mixtures of these, with an agent derived from mulch cow excrement; and method of treating growth media such as sewage or soil to stimulate the growth of aerobic bacterial to improve the condition of sewage for disposal or availability of soil nutrients for plant growth by addition of the composition to the growth.

EP 0 027 694 A1

COMPOSITION AND METHOD
FOR STIMULATION OF AEROBIC BACTERIA

BACKGROUND OF THE INVENTION

This invention relates to a composition for stimulation of aerobic bacteria in growth media and to the treatment of growth media such as sewage or soil with that composition to improve their condition.

Disposal of sewage containing human or animal excrement through discharge into septic tanks, settling ponds or cesspools is complicated by the presence of sludge from fecal matter which slows or even blocks the disposal of liquid components by percolation and is objectionable in the use of the sewage for such agricultural purposes as irrigation. While some reduction of sludge occurs through the action of microorganisms present or added to tanks, ponds or pools, the process is slower than desired and generates noxious odors including hydrogen sulfide.

It is an object of the present invention to provide a treating agent and a method of treatment of growth media such as sewage to stimulate the growth of aerobic bacteria through addition of that agent to give a high rate of sludge reduction and to minimize the development of noxious odors in the sewage.

It is a further object to provide a method for the treatment of soil by the addition of the treating agent to stimulate the growth of aerobic bacteria to increase the rate of plant growth in the soil.

## BRIEF STATEMENT OF THE INVENTION

The composition of the present invention is a combination of an organic soil treating agent derived from milking shed wastes in which aerobic bacteria-stimulating action is obtained through coaction of that agent with added triacontanol, B vitamins or mixtures of these. Addition of the combination product to sewage in accordance with the method of the present invention reduces the development of noxious odors and improves the condition of the sewage for disposal. Also in another aspect of the present method, the combination of the soil treating agent and B vitamins without the triacontanol, when applied to soil, substantially increases the rate of plant growth over that from the application of the soil treating agent alone.

## DETAILED DESCRIPTION

I have found that growth of aerobic organisms in media such as sewage or soil is greatly stimulated by the composition of the present invention in which addition of a very small percentage of triacontanol, B vitamins or mixtures of these, profoundly alters the properties of a proprietary material whose only known use was as a soil treating agent for agricultural use to prevent or retard the build-up of salts from irrigation water. Addition of the novel composition to sewage containing animal or human excrement according to one aspect of the method of the present invention reduces sludge, minimizes the development of noxious odors and facilitates disposal of residual components by percolation or otherwise. Additionally, although the addition of triacontanol to the proprietary agent destroys or reduces the effectiveness of that agent for its use in soil treatment, when that form of the composition composed of the proprietary agent and B vitamins is applied to soil, it stimulates the aerobic organisms in the soil so that the plants can more effectively use nutrients in the soil.

The proprietary material for modification by triacontanol, B vitamins or mixtures of these is sold as "Biohumus" and is described as an almost water white, thin liquid product obtained by a first digestion of milch cow exrement with yeast under mildly acid conditions and at least one further digestion of the liquid separated from the first digestion products by the action of algae and solar radiation.

The combination of the liquid digestion product with very small amounts of triacontanol or B vitamins to give unexpectedly strong growth stimulation of aerobic organisms in sewage to impart a new sludge-reducing ability is believed to show a special interaction between these materials. That is, the liquid digestion product, the triacontanol and the B vitamins have no significant bacteria content and the amount of triacontanol or B vitamins relative to the volume of sewage treated is negligible. Accordingly, it would appear that some continuing association is established between the triacontanol and B vitamins and the liquid digestion product when they are brought together since the combination provides a continuing ability to stimulate aerobic organisms in sewage to which the combination has been added.

Extremely small amounts of triacontanol are effective to impart the new sludge reducing ability to the proprietary material. Thus, useful results are obtained with as little as about 0.1 ppm. of triacontanol based on the weight of the treating agent. There does not appear to be any upper limit on the amount of triacontanol used, but no significant improvement has been observed on amounts over about 5 ppm.

Likewise, only very small amounts of the B vitamins, thiamin, riboflavin and niacin are required for combination with the liquid digestion products to provide the sludge reducing ability. Percentages as low as from about 0.02% to about 0.15% by weight of each of these vitamin components based on the weight of the treating agent have been found satisfactory and there does not appear to be any upper limit.

It has also been found that the effectiveness of the treating agent for the treatment of sewage is improved by the combination with the liquid digestion product of both the triacontanol and the B vitamins in the above amounts.

For treatment of sewage, the treating agent is simply mixed with the sewage and allowed to act. Treatment of large volumes of sewage, e. g. washings from a milking shed, may involve feeding the treating agent into a stream of the washings at a controlled rate to mix the treating agent with the washings and discharging the mixture into a disposal pond. Supply of the treating agent is cut off when the amount introduced into the disposal pond is sufficient to give the desired results, after which supply of the washings to the pond is continued. The introduction of

0027694

-5-

further washings stirs up the pond and provides further liquid which, it is believed, is useful in maintaining the activity of the treating agent.

Effective sludge reducing action is obtained where as little as 40 ppm. of the combination product are added and a preferred range of addition is from about 40 to 4000 ppm. of the treating agent based on the weight of the sewage to be treated.

Application of the treating agent based on the combination of B vitamins and the digestion product to soil may be affected by adding the agent to the irrigation or sprinkler water supplied to a field.  Addition of as little as about ten gallons of the treating agent to the water supplied to an acre has been found to give significantly better uniformity and rate of plant growth and to contribute to plant health as indicated by the color of the foliage.

The following examples are given to aid in understanding the invention, but it is to be understood that the invention is not limited to the particular procudures, conditions or materials of the examples:

Example I

Treating agent was prepared by mixing 600 mg. of triacontanol, 3000 gallons of liquid product of digestion of milch cow exrement under mild acid condition and further digestion of liquid portions from the first digestion by the action of algae and solar radiation ("Biohumus", a proprietary product of Biohumus, Inc. of Holtville, California).

The treating agent was added over a period of 24 hours to the stream of liquid product of washdown from a milking shed in which 1400 cows were milked twice a day, and the resulting mixture was discharged into settling pond having an area of 3 acres and a depth ranging from 2 to 4 feet.

The pond initially showed a high concentration of suspended solids and sludge, but the solids had substantially disappeared when the pond was examined after two weeks and again after 4 weeks as shown by the following table.

|  | Initial | 2 weeks | 4 weeks |
| --- | --- | --- | --- |
| BOD mg/l | 19,900 | 1,200 | 370 |
| COD mg/l | 59,780 | 8,964 | 2,488 |
| Total Solids, % | 8.78 | 1.08 | .3315 |
| Suspended Solids, % | 12.31 | 1.21 | .22 |
| Hydrogen Sulfide, mg/l | −0.05 | −0.05 | X |
| Settleable Solids ml/1/hr | 975 | 200 | 48 |

Note: (−) = Less than

The Biochemical Oxygen Demand, BOD, was determined by Method 405.1 (five days, 20C.) and is an experimental bioassay-type procedure which measures the dissolved oxygen consumed during dark incubation by microbial life while assimilating and oxidizing the organic matter present. The Chemical Oxygen Demand, COD, was determined by Method 410.1 (Titrimetric, Mid Level) and determined the quantity of oxygen required to oxidize the organic matter in a water sample, under specific conditions of oxidizing agent, temperature and time.

The mechanism by which the treating agent operates to reduce sludge is not clear. The proprietary material itself and its mixture with triacontanol and vitamins have no significant bacterial content so that the mixture of the invention would appear to work through preferential stimulation of desirable aerobic microoganisms present in the sewage. The following Example II provides some indication that the treating agent affects the balance between development of aerobic and development of anaerobic bacteria since in the samples containing the treating agent there is an initial sharp increase in the count of aerobic bacteria without a corresponding increase in anaerobic bacterial over a period of four weeks and only a small falling off in the aerobic bacteria count at the end of six weeks. The foregoing explanation is given as of possible help but it is to be understood that patentability is not based on its correctness.

Example II

A series of samples were prepared, each comprising five pounds of wet manure from a dairy milking shed. The samples were disposed in white translucent pails and mixed with deionized water to a volume of four and one-half gallons.

The samples had a Biochemical Oxygen Demand, "BOD", of 899 mg. per l. and a Chemical Oxygen Demand, "COD", of 18,426 mg. per l.

No additions were made to a first sample serving as a control.

A second sample had 17.7 ml. of the digestion product (Biohumus) and 0.025 mg. of triacontanol admixed; and a third sample had 17.7 ml. of the digestive product, 0.025 mg. of triacontanol, 12.5 mg of thiamine, 6.25 mg of riboflavin, 12.5 mg. of niacin and 125 mg. of magnesium cloride admixed.

The pails containing the samples were set on a roof for exposure to sunlight which aproximated 75% of daylight.

Bacterial counts were made on the samples and results obtained as recorded in the following table:

|  | Initial | 19th Day | 28th Day | 41st Day |
|---|---|---|---|---|
| Sample #1 |  |  |  |  |
| Standard Plate Count, 32° C., per ml. | not run | 1,900,000 | 360,000 | 1,000,000 |
| Coliform per 100 ml. | not run | Less than 10 | Less than 10 | Less than 10 |
| Sample #2 |  |  |  |  |
| Standard Plate Count, 32° C., per ml. | not run | 5,700,000 | 7,000,000 | 4,600,000 |
| Coliform per 100 ml. | not run | Less than 10 | Less than 10 | Less than 10 |

Sample #3

   Standard Plate

   Count, 32° C.,

   per ml.       not run   7,300,000   10,000,000  6,000,000

Coliform per                        Less than  Less than

   100 ml.      not run        20  10         10

The above results show that the combination of the digestion product and triacontanol very markedly increases the development of aerobic bacteria over the development in the control, but without corresponding increase in coliform bacteria. Also the combination of the digestion product, triacontanol, vitamins and magnesium chloride was even more effective.

The limited fall-off in the plate count at the last observation still leaves the count higher than the control, but does not appear to occur in the full scale operation as in a dairy settling pond where there is a continuing replenishment of water and sewage.

Example III

Treating agent according to the present invention was prepared by mixing a 12.5 mg. of thiamin, 6.25 mg. of riboflavin, 12.5 mg. of niacin and 125 mg. of magnesium cloride with 17.7 ml. of the liquid product of digestion of milch cow excrement with yeast under mild acid conditions and further digestion of liquid portions by the action of algae and solar radiation ("Biohumus").

A second treating agent according to the invention was prepared by mixing 17.7ml. of the digestion product (Biohumus), 0.025 mg. of triacontanol, 12.5 mg. of thiamin, 6.25 mg. of riboflavin, 12.5 mg. of niacin and 125 mg. of magnesium chloride.

A first sample was prepared comprising five pounds of wet manure from a dairy milking shed to which the treating agent of the invention was added and this material was disposed in a white translucent pail and mixed with tap water to a volume of 4-1/2 gallons.

A second sample was prepared in which the second treating agent was added, disposed in a white translucent pail and mixed with tap water to a volume of 4-1/2 gallons.

The pails containing the samples were set under a skylight for exposure to sunlight which approximated 50% of summer daylight.

Bacterial counts made on the samples are recorded in the following table:

|  | Initial | 10th Day | 23rd Day |
|---|---|---|---|
| Sample #1 |  |  |  |
| Standard Plate Count, 32° C., per ml. | 320,000 | 15,000,000 | 130,000,000 |
| Sample #2 |  |  |  |
| Standard Plate Count, 32° C., per ml. | 310,000 | 20,000,000 | 140,000,000 |

The above results show that the combination of the liquid digestion product and B vitamins according to the present invention was nearly as effective in increasing the development of aerobic bacteria was the combination of the liquid digestion product, triacontanol and B vitamins of the co-pending application.

Example IV

A further batch of treating agent of the present invention was prepared as in Example III. This treating agent was mixed with irrigation water and supplied to a portion of a field at the rate of 10 gallons of treating agent for each acre of that portion of the filed. Lettuce was then planted in the treated portion.

The digestion product (Biohumus) alone was supplied to a further portion of the field by addition to the irrigating water at the rate of 10 gallons per acre and this portion of the field was also planted with lettuce.

As the plants grew in the field, it was observed that the lettuce in the portion of the field treated with the compositon of the present invention was substantially larger and more uniform than the lettuce grown in the portion of the field treated with the digestion product alone. Also, the lettuce in the field treated with the agent of the present invention had distinctly better foliage color than the lettuce in the other portion of the field.

## CLAIMS

1. A composition for addition to growth media to stimulate the growth of aerobic bacteria comprising the product of combining (1) the liquid product of a first digestion of milch cow excrement under mild acid conditions and further digestion of liquid portions from the first digestion by the action of algae and solar radiation and (2) a member of the group consisting of triacontanol, B vitamins and mixtures of these in amount to activate an aerobic bacteria-stimulating action.

2. A composition as defined in claim 1, in which said triacontanol is present in amount of at least 0.1 ppm. based on the weight of said liquid digestion product.

3. A composition as defined in claim 1, comprising B vitamins.

4. A composition as defined in claim 3, in which said B vitamins are present in amount of from about 0.02% to about 0.15% by weight of each of thiamin, riboflavin and niacin based on the weight of said liquid digestion product.

5. A composition as defined in claim 4, in which said triacontanol is present in amount of from 0.1 ppm. to about 5 ppm.

6. A method for the treatment of growth media to stimulate the growth of aerobic bacteria therein comprising mixing with said media a treating agent comprising the product of combining (1) the liquid product of a first digestion of milch cow excrement under mild acid conditions and further digestion of liquid portions from the first digestion by the action of algae and solar radiation and (2) a member of the group consisting of triacontanol, B vitamins and mixtures of these in amount to activiate an aerobic bacteria-stimulating action.

7. A method as defined in claim 6, in which said growth medium is sewage containing excrement and in which at least 40 ppm. of the combination product based on the weight of said sewage is mixed with said sewage to accelerate reduction of sludge to soluble or finely dispersed state and to minimize generation of hydrogen sulfide.

8. A method as defined in claim 7, in which triacontanol is present in said combination product in amount of at least 0.1 ppm. based on the weight of said liquid digestion product.

9. A method as defined in claim 7, in which said B vitamins are present in amount of from about 0.02% to about 0.15% by weight of each of thiamin, riboflavin and niacin based on the weight of said liquid digestion product.

10. A method as defined in claim 9, in which said triacontanol is present in amount of from about 0.1 ppm. to about 5 ppm. based on the weight of said liquid digestion product.

11. A method as defined in claim 6, in which said growth medium is soil said treating agent is the product of combining said liquid digestion product and B vitamins and in which at least about ten gallons of the combined product is applied per acre of soil.

12. A method for treatment of soil as defined in claim 11, in which said digestion product is combined with from about 0.02% to about 0.15% by weight of each of thiamin, riboflavin and niacin based on the weight of said digestion product.

# EUROPEAN SEARCH REPORT

**European Patent Office**

| DOCUMENTS CONSIDERED TO BE RELEVANT | | | CLASSIFICATION OF THE APPLICATION (Int Cl.³) |
|---|---|---|---|
| Category | Citation of document with indication, where appropriate, of relevant passages | Relevant to claim | |
| | US - A - 3 732 089 (CH.K. MEGRO-NIGLE)  * Column 9, claim 1; column 10, claim 3; column 3, lines 7-14, 22-27 * | 1,6,7, 11 | C 02 F 3/34 C 12 N 1/38 C 05 F 13/00 |
| | FR - A - 2 375 825 (THE BOARD OF TRUSTEES)  * Page 31, claims 1,6 * | 1,2,6, 11 | |
| | BE - A - 551 877 (HOFFMANN LA ROCHE)  * Page 5, claims 1,2 * | 1,3,4, 6 | TECHNICAL FIELDS SEARCHED (Int. Cl³) |
| | DE - B - 1 301 759 (THE BRITISH COKE RESEARCH)  * Column 5, claims 1,2 * | 1,3,6, 7 | C 02 F 3/34 C 12 N 1/38 C 05 F 13/00 |
| A | CH - A - 303 261 (AKTIESELSKABET DANSK GAERINGS-INDUSTRI) | | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 20-01-1981 | TEPLY |

EPO Form 1503.1  06.78